# EUROPEAN PATENT APPLICATION

(11) **EP 2 190 008 A1**
(43) Date of publication of application: **26.05.2010**
(21) Application number: 09172865.9
(22) Date of filing: 13.10.2009
(51) Int. Cl.: H01L 21/60, B06B 1/06, G10K 11/00

(54) **Probe for ultrasound system and method of manufacturing the same**

(30) Priority: 19.11.2008 KR 20080115410
(71) Applicant: MEDISON CO., LTD., Kangwon-do 250-870 (KR)
(72) Inventor: Lee, Sung Jae, Seoul (KR); Park, Jung Lim, Seoul (KR); Kim, Jae Yk, Seoul (KR)
(74) Representative: Schmid, Wolfgang

(57) **Abstract**

A probe (100) for an ultrasound system, and a method of manufacturing the same are disclosed. The probe (100) includes a backing layer (110), a piezoelectric member (120) installed to the backing layer (110), and a unidirectional conduction part (130) installed to at least one of the backing layer (110) and the piezoelectric member (120). The probe (100) is manufactured by connecting the piezoelectric member (120) to PCBs (140) via the unidirectional conduction part (130), instead of soldering which requires difficult and laborious operations, thereby facilitating manufacture of the probe (100) while reducing an operation time in manufacture of the probe (100).

## Description

### 1. Field of the Invention

The present invention relates to a probe and, more particularly, to a probe for an ultrasound system that generates internal images of a patient body with ultrasound waves, and a method of manufacturing the same.

### 2. Description of the Related Art

Generally, an ultrasound system refers to a non-invasive apparatus that irradiates an ultrasound signal from a surface of a patient body towards a target internal organ beneath the body surface and obtains an image of a monolayer or blood flow in soft tissue from information in the reflected ultrasound signal (ultrasound echo-signal). The ultrasound system has been widely used for diagnosis of the heart, the abdomen, the urinary organs, and in obstetrics and gynecology due to various merits such as small size, low price, real-time image display, and high stability through elimination of any radiation exposure, as compared with other image diagnostic systems, such as X-ray diagnostic systems, computerized tomography scanners (CT scanners), magnetic resonance imagers (MRIs), nuclear medicine diagnostic apparatuses, and the like.

Particularly, the ultrasound system includes a probe which transmits an ultrasound signal to a patient body and receives the ultrasound echo-signal reflected therefrom to obtain the ultrasound image of the patient body.

The probe includes a transducer, a case with an open upper end, a cover coupled to the open upper end of the case to directly contact the body surface of the patient, and the like.

The transducer includes a piezoelectric layer in which a piezoelectric material converts electrical signals into sound signals or vice versa while vibrating, a coordination layer reducing a difference in sound impedance between the piezoelectric layer and a patient body to allow as much of the ultrasound waves generated from the piezoelectric layer to be transferred to the patient body as possible, a lens layer focusing the ultrasound waves, which travel in front of the piezoelectric layer, onto a predetermined point, and a backing layer blocking the ultrasound waves from traveling in a rearward direction of the piezoelectric layer to prevent image distortion.

The piezoelectric layer includes a piezoelectric member and electrodes provided to upper and lower ends of the piezoelectric member, respectively. Further, a printed circuit board (PCB) is bonded to the piezoelectric layer. The PCB is joined to the piezoelectric member by soldering with a solder such as lead or the like.

Here, since soldering between the piezoelectric member and the PCB is a difficult and laborious operation entailing heat generation, not only does the probe require a long manufacturing time, but also is likely to undergo deterioration in performance of the piezoelectric member resulting from the heat generated during the soldering operation. Moreover, since the soldering is carried out by a manual operation, a soldered portion has a low durability and uniformity, causing deterioration in performance of the probe. Therefore, there is a need for an improved probe that overcomes such problems.

### SUMMARY OF THE INVENTION

The present invention is conceived to solve the problems of the conventional technique as described above, and an aspect of the present invention is to provide an improved probe for an ultrasound system, which permits easy manufacture while preventing performance deterioration resulting from heat generation or defective connection between a piezoelectric member and a PCB during manufacturing, and a method of manufacturing the same.

In accordance with an aspect of the present invention, a probe for an ultrasound system includes a backing layer; a piezoelectric member installed to the backing layer; and a unidirectional conduction part installed to at least one of the backing layer and the piezoelectric member.

The piezoelectric member may be formed with first and second electrodes, the unidirectional conduction part being installed to the first and second electrodes.

The piezoelectric member may include a plurality of piezoelectric members arranged side by side, the unidirectional conduction part being installed to the plurality of piezoelectric members.

The unidirectional conduction part may include an anisotropic conduction material.

The probe may further include a printed circuit board (PCB) installed to the unidirectional conduction part.

In accordance with another aspect of the present invention, there is provided a method of manufacturing a probe for an ultrasound system, including: installing a piezoelectric member having first and second electrodes to a backing layer; and installing a unidirectional conduction part to the first and second electrodes.

The step of installing a piezoelectric member may include installing a plurality of piezoelectric members.

The step of installing a unidirectional conduction part may include installing the unidirectional conduction part to the plurality of piezoelectric members.

The method may further include installing a printed circuit board (PCB) to the unidirectional conduction part.

According to the embodiment of the present invention, the probe is manufactured by connecting the piezoelectric member to the PCB via the unidirectional conduction part, instead of soldering which requires difficult and laborious operations, thereby facilitating manufacture of the probe while reducing an operation time in manufacture of the probe.

Further, the first and second electrodes, which are separated from other first and second electrodes so as to form each channel, are firmly and uniformly connected to line electrodes of the PCB via the unidirectional conduction part in a single heating and pressing operation instead of the laborious soldering operation, thereby preventing performance deterioration or malfunction of the probe resulting from low durability and non-uniformity of a connected part.

### BRIEF DESCRIPTION OF THE DRAWINGS

The above and other aspects, features and advantages of the present invention will become apparent from the following description of exemplary embodiments given in conjunction with the accompanying drawings, in which:
- Fig. 1: is a perspective view of a probe for an ultrasound system according to an embodiment of the present invention;
- Fig. 2: is a flowchart of a method of manufacturing a probe for an ultrasound system according to an embodiment of the present invention; and
- Figs. 3: to 5 are views illustrating a process of installing a PCB to a piezoelectric member.

### DETAILED DESCRIPTION OF THE EMBODIMENT

Exemplary embodiments of the present invention will now be described in detail with reference to the accompanying drawings. It should be noted that the drawings are not to precise scale and may be exaggerated in thickness of lines or size of components for descriptive convenience and clarity only. Furthermore, terms used herein are defined by taking functions of the present invention into account and can be changed according to the custom or intention of users or operators. Therefore, definition of the terms should be made according to overall disclosures set forth herein.

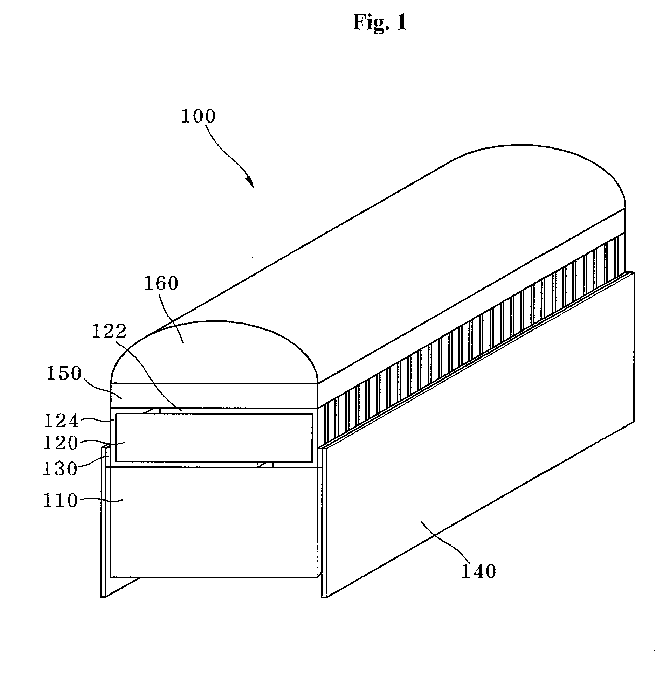

Referring to Fig. 1, which is a perspective view of a probe 100 for an ultrasound system according to an embodiment of the present invention, the probe 100 includes a backing layer 110 and a piezoelectric member 120.

The backing layer 110 is disposed at the rear of the piezoelectric member 120. The backing layer 110 reduces a pulse width of an ultrasound wave by suppressing free vibration of the piezoelectric member 120, and prevents image distortion by blocking unnecessary propagation of the ultrasound wave in the rearward direction of the piezoelectric member 120. The backing layer 110 can be formed of a material containing a rubber to which epoxy, tungsten powder, and the like are added.

The piezoelectric member 120 is "installed" to the backing layer 110. The piezoelectric member 120 generates ultrasound waves using a resonance phenomenon. The piezoelectric member 120 may be formed of a ceramic of lead zirconate titanate (PZT), a PZNT single crystal made of a solid solution of lead zinc niobate and lead titanate, a PZMT single crystal made of a solid solution of lead magnesium niobate and lead titanate, or the like.

The piezoelectric member 120 is formed with first and second electrodes 122 and 124. The first and second electrodes 122 and 124 are disposed to surround the piezoelectric member 120. The first and second electrodes 122 and 124 may be formed of a highly conductive metal such as gold, silver or copper. Here, one of the first and second electrodes 122 and 124 serves as a positive pole of the piezoelectric member 120, and the other serves as a negative pole of the piezoelectric member 120. The first and second electrodes 122 and 124 are separated from each other to allow the positive pole and the negative pole to be separated from each other. In this embodiment, the first and second electrodes 122 and 124 are illustrated as serving as the positive and negative poles, respectively.

Further, the first and second electrodes 122 and 124 are configured to be disposed symmetrically to each other, thereby making upper and lower portions of the piezoelectric member 120 symmetrical to each other. Herein, each of the first and second electrodes 122 and 124 may have a "J"-shape that surrounds the piezoelectric member 120. With the first and second electrodes 122 and 124 disposed on the piezoelectric member 120, the upper and lower portions of the piezoelectric member 120 are symmetrical to each other, so that there is no need for differentiating the upper and lower portions of the piezoelectric member 120, thereby allowing the piezoelectric member 120 to be installed to the backing layer 110 without differentiating the upper and lower portions thereof.

An array of piezoelectric members 120 with the configuration described above are arranged to form multiple channels. According to this embodiment, the piezoelectric member 120 is divided into the plural piezoelectric members 120 separated a predetermined distance from each other on a single backing layer 110 by dicing, and the plural piezoelectric members 120 are arranged side by side to constitute the array of piezoelectric members 120. However, the present invention is not limited to this configuration. Alternatively, both the piezoelectric member 120 and the backing layer 110 may be divided into plural piezoelectric members 120 and plural backing layers 110 separated a predetermined distance from each other by dicing, such that plural laminates of the backing layers 110 and the piezoelectric members 120 may be disposed side by side in an array.

The probe 100 for an ultrasound system according to this embodiment may further include a unidirectional conduction part 130 and PCBs 140.

The unidirectional conduction part 130 is installed to the piezoelectric members 120 which are disposed in an array as described above. A single unidirectional conduction part 130 comprising an anisotropic conduction material is installed to each side of the first and second electrodes 122 and 124.

The anisotropic conduction material is a bonding material which can accomplish electrical and mechanical coupling between electrodes by application of a predetermined pressure and heat thereto. The anisotropic conduction material has properties dependent on the application direction of pressure, so that only a part of the anisotropic conduction material exposed to pressure exhibits electrical conductivity, but other parts thereof free from the pressure do not exhibit the electrical conductivity. Thus, the unidirectional conduction part 130 comprising the anisotropic conduction material allows separation of electrodes between channels in a single mechanical process.

The PCBs 140 are installed to the unidirectional conduction part 130. The PCBs 140 are disposed substantially perpendicular with respect to the direction in which the backing layer 110 and the piezoelectric member 120 are laminated. The PCB 140 includes a flexible printed circuit board (FPCB), and any other configurations capable of supplying signals or electricity.

According to this embodiment, the PCB 140 having a plurality of line electrodes (not shown) formed thereon is installed to each side of the first and second electrodes 122 and 124. The PCBs 140 are connected to the piezoelectric members 120 via the unidirectional conduction part 130.

Herein, the term "installing or installed" means that two or more components are electrically connected to each other through interconnection therebetween. Hence, the PCBs 140 are electrically connected to the piezoelectric members 120 through interconnection therewith, so that the PCBs 140 can be installed to the piezoelectric members 120.

In other words, when the PCBs 140 are compressed at a predetermined pressure and heat with the unidirectional conduction part 130 interposed therebetween, each of the PCBs 140 is mechanically coupled to the piezoelectric members 120 via the unidirectional conduction part 130 while plural line electrodes of the PCBs 140 are electrically connected to the first and second electrodes 122 and 124 of the piezoelectric members 120. A detailed description of this configuration will be described below.

On the other hand, reference numerals 150 and 160 indicate a coordination layer of a glass or resin material for reducing a difference in sound impedance between a patient body and the probe, and a lens layer for focusing ultrasound waves traveling in front of the piezoelectric member 120 onto a particular point, respectively.

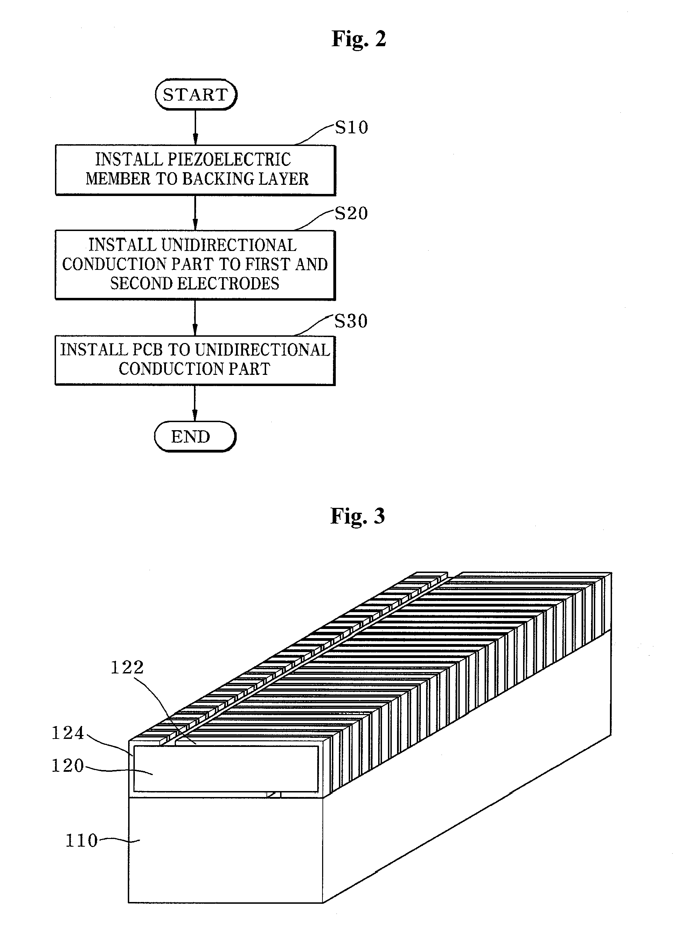

Fig. 2 is a flowchart of a method of manufacturing a probe for an ultrasound system according to an embodiment of the present invention, and Figs. 3 to 5 are views illustrating a process of installing a PCB to a piezoelectric member.

Referring to Figs. 2 to 5, a method of manufacturing a probe for an ultrasound system according to an embodiment of the present invention will now be described.

To manufacture a probe 100 for an ultrasound system according to the embodiment of the invention, first, a backing layer 110 is formed using a material including a rubber, to which epoxy resin or tungsten powder is added, and a piezoelectric member 120 having first and second electrodes 122 and 124 is installed to the backing layer 110 in S10.

Here, the first and second electrodes 122 and 124 are formed symmetrically to each other in a "J"-shape surrounding the piezoelectric member 120, so that the upper and lower portions of the piezoelectric member 120 become symmetrical to each other to thereby eliminate a need for differentiating the upper and lower portions of the piezoelectric member 120. Accordingly, the piezoelectric member 120 can be installed to the backing layer 110 without differentiating the upper and lower portions of the piezoelectric member 120, thereby allowing easy manufacture of the probe 100.

On the other hand, the piezoelectric member 120 is divided into a plurality of piezoelectric members 120 separated a predetermined distance from each other to constitute an array of piezoelectric members 120 arranged side by side, so that the array of piezoelectric members 120 can be used as multiple channels corresponding to a plurality of line electrodes formed on a PCB 140.

A unit of the separated piezoelectric member 120 constitutes a single channel. Thus, such units of the piezoelectric members 120 are arranged side by side in an array, thereby constituting multiple channels.

According to this embodiment, a laminate of the backing layer 110 and the piezoelectric member 120 is diced by a dicing apparatus. Dicing is performed to a sufficient depth to allow each of the first and second electrodes 122 and 124 to be reliably divided into plural electrodes.

By dicing, the piezoelectric member 120 is divided into the plural piezoelectric members 120 separated a predetermined distance from each other such that the first electrode 122 and the second electrode 124 formed on a single separated piezoelectric member 120 can be completely electrically separated from the first electrode 122 and the second electrode 124 on another adjacent piezoelectric member 120.

According to this embodiment, only the piezoelectric member 120 is illustrated as being divided by dicing to constitute the array of piezoelectric members 120 arranged side by side on the single backing layer 110. However, it should be noted that the present invention is not limited to this configuration. Alternatively, the backing layer 110 may also be divided along with the piezoelectric member 120 by dicing to divide the laminate of the backing layer 110 and the piezoelectric member 120 into plural laminates of the backing layers and the piezoelectric members such that an array of separated laminates arranged side by side can be constituted.

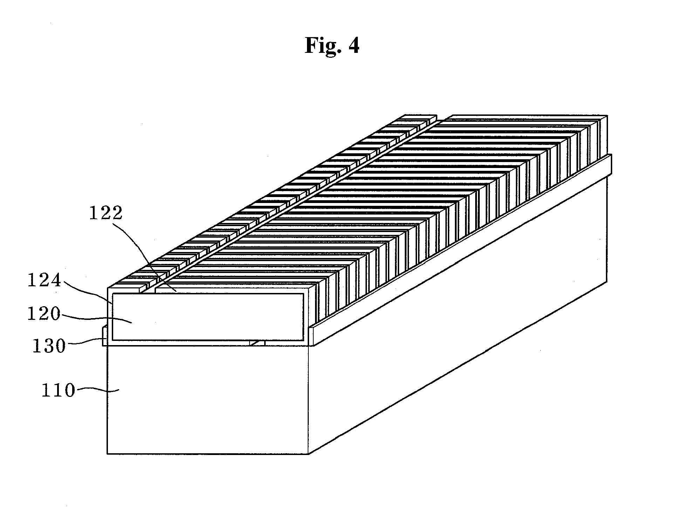

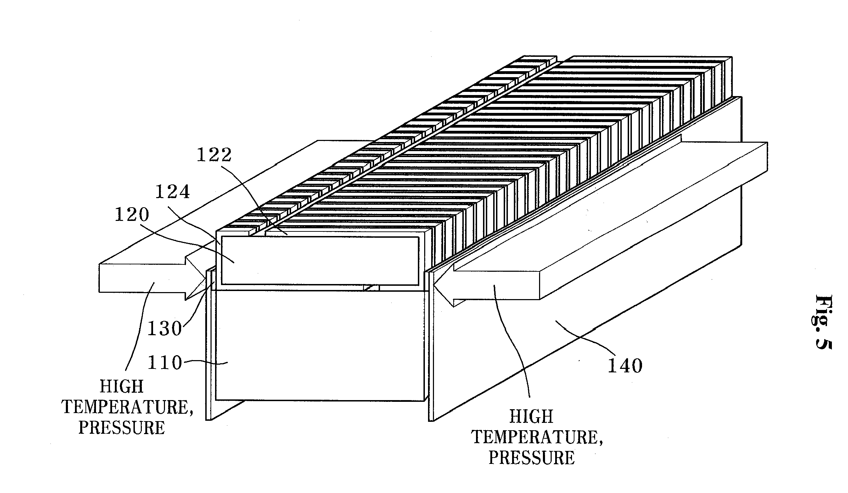

After the piezoelectric members 120 are installed to the backing layer 110, in S20, a unidirectional conduction part 130 comprising an anisotropic material is installed to the plural first and second electrodes 122 and 124, which are arranged side by side in an array, and PCBs 140 are installed to the unidirectional conduction part 130 disposed on the first and second electrodes 122 and 124 in S30, as shown in Figs. 4 and 5. At this time, the unidirectional conduction part 130 and PCBs 140 are provided substantially perpendicular with respect to the direction of laminating the backing layer 110 and the piezoelectric members 120.

The anisotropic conduction material is a bonding material which can accomplish electrical and mechanical coupling between electrodes by application of predetermined pressure and heat thereto. The anisotropic conduction material contains conductive particles in a predetermined density to provide anisotropic conductivity. That is, the conductive particles of the anisotropic conduction material become nonconductive when pressure is not applied thereto. However, when pressure is applied thereto, the conductive particles of the anisotropic conduction material are brought into contact with each other and exhibit conductivity only in the direction in which pressure is applied.

Therefore, when a predetermined pressure and heat are applied to the unidirectional conduction part 130 via the PCBs 140 with the unidirectional conduction part 130 interposed between the PCBs 140 and the plural piezoelectric members 120 arranged side by side, and with the PCBs 140 aligned to allow the respective first and second electrodes 122 and 124 to be connected to the associated line electrodes of the PCBs 140, the PCBs 140 per se are bonded to the piezoelectric members 120 via the unidirectional conduction part 130, and the line electrodes of the PCBs 140 are electrically connected to the first and second electrodes 122 and 124 via the unidirectional conduction part 130, respectively.

At this time, the pressure applied to the unidirectional conduction part 130 acts on connected parts between the first and second electrodes 122 and 124 and the line electrodes, so that the piezoelectric members 120 and the line electrodes of the PCBs 140 are connected to each other to provide conductivity only in each channel.

On the other hand, although the method of manufacturing the probe has been illustrated as performing the operation of installing the unidirectional conduction part 130 and the PCBs 140 after the operation of installing the piezoelectric member 120 to the backing layer 110 in this embodiment, the present invention is not limited to this order. In other words, these operations may be performed in a reverse sequence or at the same time.

In this embodiment, the unidirectional conduction part 130 is illustrated as being installed to the piezoelectric members 120, but the present invention is not limited to this configuration. Alternatively, the unidirectional conduction part 130 may be installed to the backing layer 110, in which electrodes connected to the first and second electrodes 122 and 124 of the piezoelectric members 120 for the respective channels are formed, such that the electrodes of the backing layer 110 can be electrically connected to the PCBs 140 therethrough.

In the probe 100 for an ultrasound system according to the embodiment of the invention as described above, the piezoelectric members 120 are electrically connected to the PCBs 140 by electrically connecting the first and second electrodes 122 and 124 to the line electrodes of the PCBs 140 via the unidirectional conduction part 130, thereby providing the following advantageous effects.

First, in manufacture of the probe 100, the piezoelectric members 120 and the PCBs 140 are connected to each other via the unidirectional conduction part 130 instead of soldering which requires difficult and laborious operations, thereby facilitating manufacture of the probe while reducing an operation time in manufacture of the probe.

Secondly, the first and second electrodes 122 and 124, which are separated from other first and second electrodes so as to form each channel, are firmly and uniformly connected to the line electrodes of the PCBs 140 via the unidirectional conduction part 130 in a single heating and pressing operation instead of the laborious soldering, thereby preventing performance deterioration or malfunction of the probe resulting from low durability and non-uniformity of connection there between.

Although the present invention has been described with reference to the embodiments shown in the drawings, it will be apparent to those skilled in the art that the embodiments are given by way of illustration only, and that various modifications and equivalent embodiments can be made without departing from the spirit and scope of the present invention. Accordingly, the scope of the present invention should be limited only by the accompanying claims.

## Claims

1. A probe for an ultrasound system including a backing layer (110) and a piezoelectric member (120), **characterized by** comprising:
a backing layer (110);
a piezoelectric member (120) installed to the backing layer (110); and
a unidirectional conduction part (130) installed to at least one of the backing layer (110) and the piezoelectric member (120).

2. The probe according to claim 1, **characterized in that** the piezoelectric member (120) is formed with first and second electrodes (122, 124), the unidirectional conduction part (130) being installed to the first and second electrodes (122, 124).

3. The probe according to claim 1, **characterized in that** the piezoelectric member (120) comprises a plurality of piezoelectric members (120) arranged side by side, the unidirectional conduction part (130) being installed to the plurality of piezoelectric members (120).

4. The probe according to any one of claims 1 to 3, **characterized in that** the unidirectional conduction part (130) comprises an anisotropic conduction material.

5. The probe according to any one of claim 1 to 3, **characterized by** further comprising a printed circuit board (PCB) (140) installed to the unidirectional conduction part (130).

6. A method of manufacturing a probe for an ultrasound system, the probe including a backing layer (110) and a piezoelectric member (120), **characterized in that** the method comprises:
installing a piezoelectric member (120) having first and second electrodes (122, 124) to a backing layer (110); and
installing a unidirectional conduction part (130) to the first and second electrodes (122, 124).

7. The method according to claim 6, **characterized in that** the step of installing a piezoelectric member (120) comprises installing a plurality of piezoelectric members (120).

8. The method according to claim 6, **characterized in that** the step of installing a unidirectional conduction part (130) comprises installing the unidirectional conduction part (130) to the piezoelectric member (120), the piezoelectric member (120) comprising a plurality of piezoelectric members (120).

9. The method according to any one of claims 6 to 8, **characterized in that** the method further comprises installing a PCB (140) to the unidirectional conduction part (130).
